# EUROPÄISCHE PATENTSCHRIFT

(11) **EP 1 933 695 B1**
(45) Veröffentlichungstag und Bekanntmachung des Hinweises auf die Patenterteilung: **27.05.2015**
(21) Anmeldenummer: 06806286.8
(22) Anmeldetag: 15.10.2006
(51) Int. Cl.: A61B 5/00, A61B 5/151, G01N 21/77, A61B 5/1459

(54) **TESTELEMENT UND TESTSYSTEM ZUR UNTERSUCHUNG EINER KÖRPERFLÜSSIGKEIT**
TEST ELEMENT AND TEST SYSTEM FOR EXAMINING A BODY FLUID
ELEMENT D'ANALYSE ET SYSTEME D'ANALYSE POUR L'ANALYSE D'UN LIQUIDE CORPOREL

(30) Priorität: 15.10.2005 EP 05022535
(43) Veröffentlichungstag der Anmeldung: 25.06.2008
(60) Teilanmeldung: 14191436.6
(73) Patentinhaber: F. Hoffmann-La Roche AG, 4002 Basel (CH); Roche Diagnostics GmbH, 68305 Mannheim (DE)
(72) Erfinder: HAAR, Hans-Peter, 69168 Wiesloch (DE); LIST, Hans, 64754 Hesseneck-Kailbach (DE)
(74) Vertreter: Pfiz, Thomas
(86) Internationale Anmeldenummer: PCT/EP2006/009945
(87) Internationale Veröffentlichungsnummer: WO 2007/045412

(56) Entgegenhaltungen:
- EP-A- 1 441 214
- EP-A2- 0 221 005
- WO-A-97/42882
- WO-A-2004/110275
- WO-A-2005/104949
- WO-A2-2005/084530
- DE-B3- 10 325 699
- JP-A- 2005 087 613
- US-A- 4 622 974
- US-A1- 2003 083 686
- US-A1- 2005 059 166
- US-B1- 6 584 335

## Beschreibung

Die Erfindung betrifft ein Testelement als Einmalartikel zur Untersuchung einer Körperflüssigkeit mit einem in ein Körperteil einstechbaren Stechteil, einem daran ausgebildeten Sammelbereich für durch den Einstich erhaltene Körperflüssigkeit und mindestens einem Lichtleiter für eine optische Messung in dem Sammelbereich, der als kapillaraktiver Kanal durch eine in Stechrichtung sich erstreckende Sammelausnehmung des Stechteils gebildet ist, wobei der verschiebefest in das Stechteil integrierte Lichtleiter mit seinem distalen Ende in einer proximalen Messzone der Sammelausnehmung angeordnet ist. Die Erfindung betrifft auch ein Testsystem zum Einsatz solcher Testelemente.

Bei in der Regel täglich mehrfach durchgeführten Blutzucker-Selbstkontrollen im Rahmen einer Insulinbehandlung ist es wünschenswert, der betroffenen Person möglichst wenige Handhabungsschritte aufzuerlegen und zugleich eine schmerzarme und zuverlässige Messung sicherzustellen. Dabei werden aus hygienischen Gründen Einwegartikel eingesetzt, die als Massenprodukt möglichst günstig herstellbar sein sollen. Die herkömmlichen Konzepte sehen einen Transport von Blutflüssigkeit, die üblicherweise durch Lanzetteneinstich aus dem Fingergewebebereich entnommen wird, auf ein gesondertes sensorisches Element beispielsweise in Form eines Teststreifens vor, um dann die eigentliche Messprozedur zu starten.

Für eine weitergehende Systemintegration wurde in der DE 10325699 B3 bereits eine kombinierte Anordnung vorgeschlagen, in der eine lichtleitende Hohlfaser konzentrisch um eine koaxial verschiebliche Lanzette angeordnet ist. Die Hohlfaser weist an einer distalen Stirnseite eine Reagenzschicht auf, die beim Einstechen der Lanzette in das Körperteil mit austretendem Blut beaufschlagt wird, während in das proximale Hohlfaserende das Licht einer Analyseeinheit ein- bzw. ausgekoppelt werden kann. Bei einer komplementären Ausführung weist das System eine Hohlkanüle mit einem darin verschieblichen Lichtleiter auf, dessen in der Kanülenöffnung angeordnetes distales Ende ebenfalls mit einem analytspezifischen Reagenz beschichtet ist. Um die Probenaufnahme zu erleichtern, wird nach dem Stechvorgang das Lichtleiterende aus der Kanülenöffnung geschoben, bis es darüber hinausragt und somit für einen problemlosen Kontakt des Testfelds mit dem Blut des Patienten sorgt. Für die Relativverschiebung von Stechelement und lichtleitendem Element ist allerdings eine zusätzliche Antriebssteuerung erforderlich, wobei eine Erfolgskontrolle des Blutkontakts nicht vorgesehen ist.

Ein Testelement nach dem Oberbegriff des Anspruchs 1 ist aus der Druckschrift JP 2005087613 bekannt.

Ausgehend hiervon liegt der Erfindung die Aufgabe zugrunde, die im Stand der Technik bekannten Systeme weiter zu verbessern, insbesondere die Nachteile eines aufwändigen Probentransports zu vermeiden und mit zuverlässigen Mitteln die Bedienerfreundlichkeit zu erhöhen.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, die Flüssigkeit in einer kurzen kapillaren Sammelstrecke selbsttätig und weitgehend totvolumenfrei zu gewinnen und daraus ein optisches Messignal abzuleiten. Dementsprechend wird erfindungsgemäß vorgeschlagen, die Sammelausnehmung als ein- oder beidseitig offener Schlitz ausgebildet ist, und dass das distale Ende des Lichtleiters stirnseitig mit einem fest aufgebrachten Testfeld für einen Analyten in der Körperflüssigkeit versehen ist, wobei das Testfeld quer zu der Längserstreckung der Sammelausnehmung ausgerichtet ist, so dass die Körperflüssigkeit frontal gegen das Testfeld anströmt. In der Sammelausnehmung kann eine (möglichst geringe) Blutmenge aufgenommen werden, so dass die Untersuchung nicht durch störende Gewebebestandteile in der Stichwunde und/oder durch unkontrollierbare mechanische Druckbelastungen interferiert wird. Durch den verschiebefesten Einbau des mindestens einen Lichtleiters wird eine auch herstellungstechnisch vereinfachte Integration erreicht, wobei die Anordnung in der proximalen Zone eine Erfolgskontrolle des Sammelvorgangs erlaubt.

Um eine zuverlässige Messung zu gewährleisten, sieht eine vorteilhafte Ausgestaltung vor, dass der Lichtleiter mit seinem distalen Ende einen Fühler zur Detektion von die Sammelausnehmung füllender Körperflüssigkeit bildet. Um die hinreichende Befüllung des Sammelvolumens zu gewährleisten, ist es vorteilhaft, wenn der Lichtleiter in der Endphase der Flüssigkeitsaufnahme in der Sammelausnehmung einen Körperflüssigkeitskontakt erfasst.

Ergänzend zu einer optischen Detektion ist es auch möglich, dass mindestens eine mit einer elektrischen Spannung beaufschlagbare Elektrode für eine Flüssigkeitskontakterfassung oder eine Analyterfassung im Bereich der Sammelausnehmung angeordnet ist, wobei die Elektrode durch einen in das Stechteil integrierten elektrischen Leiterdraht gebildet sein kann.

Eine weitere Verbesserung ergibt sich dadurch, dass der Lichtleiter und/oder die Elektrode ein Messsignal zur Bewegungssteuerung des Stechteils in Abhängigkeit von einem erfassten Körperflüssigkeitskontakt liefert.

Gemäß einer bevorzugten Ausgestaltung ist im Bereich des distalen Endes des Lichtleiters ein auf einen Analyten in der Körperflüssigkeit ansprechendes, insbesondere irreversibel chemisch reagierendes Testfeld angeordnet. Eine weitere Miniaturisierung ist dadurch möglich, dass das distale Ende des Lichtleiters stirnseitig mit einem vorzugsweise als Reagenzschicht fest aufgebrachten Testfeld für einen Analyten in der Körperflüssigkeit versehen ist.

Um die Zuverlässigkeit gerade bei der Untersuchung von durch den Benutzer nicht mehr wahrnehmbaren Kleinstmengen weiter zu erhöhen, ist es von Vorteil, wenn mindestens zwei, vorzugsweise drei gesonderte Lichtleiter für eine Parallelmessung in das Stechteil integriert sind, wobei die distalen Enden der Lichtleiter im seitlichen Abstand voneinander angeordnet sind.

Für einen trotz der Miniaturisierung robusten Aufbau ist es von Vorteil, wenn die distalen Enden der Lichtleiter über eine Trägerfolie an ein gemeinsames Testfeld optisch angekoppelt sind, wobei das Testfeld durch eine auf die von den Lichtleitern abgewandte freie Frontseite der Trägerfolie aufgebrachte Reagenzschicht gebildet ist. Für eine optische Messung mit hoher Signalqualität ist ein reflektiver Strahlengang zwischen den sende- und empfangsseitigen Lichtleiterenden über das rückseitig bestrahlte Testfeld von besonderem Vorteil. Hierfür sollte die Dicke der Trägerfolie das 0,5- bis 1,5-fache des gegenseitigen Abstands der Mittellinien benachbarter Lichtleiter betragen. Eine weitere Verbesserung wird dadurch erreicht, dass die Lichtleiter, die Trägerfolie und die Reagenzschicht einen aufeinander angepassten, im Wesentlichen übereinstimmenden Brechungsindex aufweisen. Dies lässt sich dadurch optimieren, dass das Testfeld in einer Reagenzschicht reaktionsneutrale Hilfsstoffe, insbesondere Salze wie Kochsalz enthält, welche in Lösung in der Körperflüssigkeit den Brechungsindex der Reagenzschicht an den Brechungsindex des mindestens einen Lichtleiters anpassen.

Vorteilhafterweise ist der Lichtleiter vorzugsweise über optische Steckverbindungen mit einer optischen Sende- und/oder Empfangseinheit eines Messgeräts lösbar verbindbar. Dies lässt sich bevorzugt dadurch realisieren, dass das Stechteil vorzugsweise formschlüssig an eine Kopplungseinheit eines Stechantriebs ankoppelbar ist, wobei die Kopplungseinheit eine bei der Stechbewegung mitbewegbare optische oder opto-elektronische Schnittstelle für die Verbindung des Lichtleiters mit einer Analyseeinheit aufweist.

Eine weitere vorteilhafte Ausführung sieht vor, dass die Länge der Sammelausnehmung in Stechrichtung gesehen so bemessen ist, dass der proximale Endabschnitt der Sammelausnehmung beim Einstich außerhalb des Körperteils verbleibt, so dass in jedem Fall ein Körperkontakt mit Testchemie vermieden wird und umgekehrt störende Zellbestandteile ferngehalten werden können.

Um eine rasche Flüssigkeitsaufnahme zu erlauben und somit auch das Schmerzempfinden zu minimieren, ist es von Vorteil, wenn die Sammelausnehmung ein Volumen von 1 bis 100 nl, vorzugsweise 5 bis 50 nl aufweist. Hierbei ist es günstig, daß die Sammelausnehmung eine Kapillare für eine kapillaraktive Flüssigkeitsaufnahme bildet. Eine weiter verbesserte Flüssigkeitsaufnahme lässt sich dadurch erreichen, dass die Sammelausnehmung als mindestens einseitig, vorzugsweise beidseitig offener Schlitz ausgebildet ist.

Für die Positionierung der Lichtleiter ist es vorteilhaft, wenn die Sammelausnehmung einen im Querschnitt verbreiterten proximalen Endabschnitt als Messzone aufweist.

Vorteilhafterweise ist der Lichtleiter durch eine optische Faser oder ein Faserbündel gebildet. Denkbar ist es auch, dass der Lichtleiter durch eine aus einem lichtleitenden Material geformte Partie des Stechteils gebildet ist. Für eine optimierte Lichtübertragung ist es günstig, wenn der Lichtleiter an seinem proximalen Ende eine größere Querschnittsfläche aufweist als an seinem distalen Ende.

Eine herstellungstechnische Vereinfachung kann dadurch erreicht werden, dass an das Stechteil vorzugsweise durch Zweikomponenten-Spritztechnik ein Träger aus Kunststoff angeformt ist. Eine weitere fertigungstechnisch vorteilhafte Variante sieht vor, dass das Stechteil aus mehreren Flachmateriallagen gebildet ist, wobei der Lichtleiter vorzugsweise in einer Aufnahmenut zwischen zwei Flachmateriallagen angeordnet ist.

Für einen präzisen Stechvorgang ist es vorteilhaft, wenn das Stechteil ein durch einen Schliff geschärftes und/oder eine Beschichtung gehärtetes Stechorgan aufweist. Ebenso kommen in Frage laseroptische Materialbearbeitungsverfahren, insbesondere Ablationsverfahren zum Schärfen von Kanten, wie sie auch aus der Herstellung von Rasierklingen bekannt sind.

Für eine verbesserte Flüssigkeitsaufnahme kann die Sammelausnehmung mit einer hydrophilen Beschichtung versehen sein.

Ein selbsttätiger Sammelvorgang von durch einen Hauteinstich erhaltener Körperflüssigkeit in einer geringen, aber hinreichenden Menge kann dadurch erreicht werden, dass die Sammelausnehmung eine Kapillare für eine selbsttätige Befüllung mit Körperflüssigkeit bildet, wobei die Länge der Kapillare ein Mehrfaches ihres Durchmessers beträgt. Besonders bevorzugt für eine Analyse mikroskopischer Flüssigkeitsmengen ist eine Anordnung, in der das Testfeld quer zu der Längserstreckung der Sammelausnehmung ausgerichtet ist, so dass die Körperflüssigkeit frontal gegen das Testfeld anströmt.

Gegenstand der Erfindung ist auch ein Testsystem zur Untersuchung einer Körperflüssigkeit, insbesondere Blut, mit mindestens einem erfindungsgemäßen Testelement. Ein erhöhter Benutzerkomfort wird dadurch erreicht, dass eine Vielzahl von Testelementen in einem Magazin bevorratet sind.

Für den Messablauf ist es von Vorteil, wenn das Testelement durch einen Stechantrieb in einer hin- und hergehenden Stechbewegung antreibbar ist, und wenn bei der Stechbewegung Sende- und/oder Empfangsmittel einer Analyseeinheit mit dem Lichtleiter bzw. Signalleiter es Testelements koppelbar sind. Dies lässt sich dadurch verwirklichen, dass die Sende- und/oder Empfangsmittel vorzugsweise als Einbauteile eines Antriebsstößels bei der Stechbewegung mit dem Testelement mitbewegbar sind.

Bevorzugt werden derartige Testsysteme in Form eines tragbaren Handgeräts für die Blutzuckerbestimmung oder auch für andere personennahe Überwachungen beispielsweise für die Gerinnungsdiagnostik eingesetzt.

Im Folgenden wird die Erfindung anhand der in der Zeichnung schematisch dargestellten Ausführungsbeispiele näher erläutert. Es zeigen
- Fig. 1: ein Blutzuckermessgerät in einer schaubildlichen Darstellung;
- Fig. 2: das Gerät nach Fig. 1 in einer perspektivischen Ansicht;
- Fig. 3: ein mit Lichtleitern versehenes Testelement in einer Seitenansicht;
- Fig. 4: das an einen Stechantrieb angekoppelte Testelement gemäß Fig. 3;
- Fig. 5: eine abgebrochene ausschnittsweise Vergrößerung der Fig. 4;
- Fig. 6: eine weitere Ausführungsform einer Kopplungseinheit für die Lichtleiter des Testelements in einer abgebrochenen Seitenansicht;
- Fig. 7 und 8: eine weitere Ausführungsform eines Testelements in Seitenansicht und ausschnittsweisen Vergrößerungen;
- Fig. 9: ein Testelement als Folienverbundteil in Explosionsdarstellung und zusammengebaut in perspektivischer Ansicht;
- Fig. 10: einen ausschnittsweisen Längsschnitt durch ein Testelement im Bereich der Messwerterfassung an der Sammelausnehmung; und
- Fig. 11: eine weitere Ausführungsform eines Testelements mit abgekröpftem Stechteil in perspektivischer Darstellung.

Die in der Zeichnung dargestellten Testelemente 10 lassen sich als Einmalartikel für eine Blutzuckermessung in einem dafür ausgebildeten Handgerät 12 einsetzen. Zu diesem Zweck umfassen die Testelemente 10 ein in einer Stechbewegung in ein Körperteil einstechbares Stechteil 14, einen daran ausgebildeten Sammelbereich 16 zur Aufnahme einer durch den Einstich erhaltenen Blutprobe und mindestens einen Lichtleiter 18 für eine optische Messung in dem Sammelbereich 16.

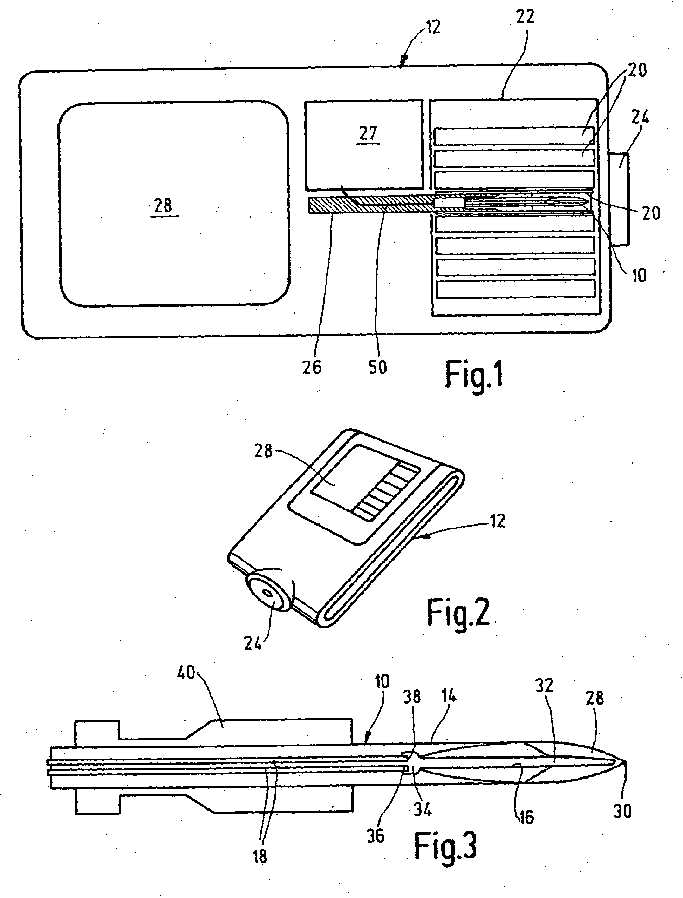

Wie in Fig. 1 veranschaulicht, sind eine Vielzahl von Testelementen 10 in jeweiligen Magazinkammern 20 eines stapel- oder trommelartigen Magazins 22 sukzessive in eine aktive Gebrauchsposition bezüglich einer mit einer Durchstechöffnung versehenen Auflage 24 zur Fingerpositionierung eines Benutzers bringbar. Ein in die Magazinkammer 20 eingreifender Stechantrieb 26 ermöglicht dabei eine hin- und hergehende Bewegung des Stechteils 14 längs einer Stechachse bzw. in Stechrichtung. Die Blutaufnahme in dem Sammelbereich 16 erfolgt dann während einer ggf. etwas in dem Körperteil zurückgezogenen Sammelstellung des Stechteils 14.

Die erfolgreiche Blutaufnahme und/oder der Gehalt eines Analyten (Glucose) darin kann über die Lichtleiter 18 gleichsam vor Ort in einem Kleinstvolumen erfasst werden, ohne dass größere Transportstrecken und Totvolumen in Kauf zu nehmen wären. Über eine Mikroprozessor-gestützte Verarbeitungseinheit 27 und Ausgabeeinheit 28 ist eine Analyse und Anzeige des momentanen Blutglukosespiegel möglich. Gemäß Fig. 2 werden alle Gerätefunktionen in einem kompakten batteriebetriebenen Handgerät realisiert, so dass die Selbstbestimmung der Blutzuckerkonzentration in einem vollautomatischen Messablauf auch für Laien mit hohem Handhabungskomfort zuverlässig durchführbar ist.

Generell können solche Messungen auch an anderen Körperteilen, beispielsweise im weniger schmerzempfindlichen Arm- oder Bauchbereich vorgenommen werden, wobei als Körperflüssigkeit für die Probennahme neben Kapillarblut aus der Haut auch Gewebeflüssigkeit oder Mischungen davon in Frage kommen.

Fig. 3 zeigt ein einzelnes Testelement 10 mit einem schaftförmiglanggestreckten Stechteil 14, an dessen distalem Ende ein durch einen Anschliff 28 geschärfte Spitze 30 als Stechorgan ausgebildet ist. Der Sammelbereich 16 ist als ein- oder beidseitig offener Längsschlitz 32 ausgeformt, der als kapillaraktiver Kanal den Transfer einer mikroskopischen Probenmenge in eine außerhalb der Haut befindliche proximale Messzone 34 ermöglicht. Zu diesem Zweck ist die Länge des Schlitzes 32 so bemessen, dass die Messzone 34 auch bei der maximalen Eindringtiefe des Stechorgans 30 noch außerhalb des Körperteils verbleibt. Zugleich kann durch die langgestreckte Schlitzöffnung eine effektive Flüssigkeitsaufnahme ohne die Gefahr einer Verstopfung durch Zellbestandteile gewährleistet werden. Die seitliche Kanalöffnung verläuft also vorteilhaft über die Länge der Sammelausnehmung 16.

Für eine möglichst schonende und schmerzarme Blutentnahme ist es vorgesehen, dass das Volumen des Sammelbereichs 16 lediglich einige 10 Nanoliter beträgt. Hierbei ist es günstig, wenn durch hydrophile Beschichtung der Sammelausnehmung 16 und ggf. Hydrophobierung der angrenzenden Bereiche dafür gesorgt wird, dass die Flüssigkeit im Wesentlichen nur dorthin gelangt, wo sie benötigt wird. Die unterschiedlichen hydrophilen und hydrophoben Bereiche können z.B. so hergestellt werden, dass Beschichtungsmaterialien im Tampondruckverfahren aufgebracht und/oder auch photochemisch funktionalisiert werden.

Wie aus Fig. 3 weiter zu ersehen ist, sind zwei parallele Lichtleiter 18 fest in das Stechteil 14 integriert, so dass die im seitlichen Abstand voneinander befindlichen distalen Lichtleiterenden 36 in die querschnittserweiterte proximale Messzone 34 des Sammelbereichs 16 hineinragen. Generell ist für eine "Online-Detektion" der erfolgreichen Blutaufnahme wichtig, dass der oder die Lichtleiter den Sammelbereich im körperabgewandten, d.h. proximalen Endbereich zumindest partiell begrenzen. Da in diesen Endabschnitt lediglich wenige Nanoliter an Blut gelangen, ist durch die Doppelerfassung mittels der parallelen Lichtleiter 18 eine höhere Messsicherheit gegeben. Dies ist für den Anwender von besonderem Nutzen, weil er nicht nur einen Messwert erhält, sondern zusätzlich eine Qualität sichernde Information aus dem Vergleich der beiden Messwerte abgeleitet werden kann.

Eine besondere Funktionalität der Lichtleiter 18 kann darin bestehen, dass deren Enden 36 als Fühler zur Detektion einer erfolgreichen Befüllung der Sammelausnehmung 16 ausgebildet sind. Speziell kann durch eine Brechungsindexänderung bei Flüssigkeitskontakt in der Endphase der Blutaufnahme ein Signal abgeleitet werden, das zur variablen Steuerung der Stechbewegung im Sinne eines erfolgreichen Sammelvorgangs nutzbar wäre. So könnte ein reguläres Sammelintervall von beispielsweise 0,1 s in Einzelfällen auch bis zu beispielsweise 1 s ausgedehnt werden, um die Messung nicht erfolglos abbrechen zu müssen. Grundsätzlich ist es auch möglich, dass ein solcher Flüssigkeitskontakt über integrierte elektrische Fühler, insbesondere eine Drahtelektrode anstelle eines Lichtleiters erfasst wird (nicht gezeigt).

Die Lichtleiter 18 können durch eine einzelne Faser oder ein Faserbündel gebildet sein. Dabei kann auch eine so genannte gezogene Faser verwendet werden, deren proximaler Querschnitt kleiner ist als der distale Querschnitt, so dass eine gute optische Verbindung an einer Kopplungsstelle gewährleistet ist.

Zur direkten Erfassung des Analyten in der Messzone 34 sind die distalen Enden 36 der Lichtleiter 18 stirnseitig mit einer Reagenzschicht als Testfeld 38 beschichtet. Die Testchemie als an sich bekanntes enzymatisches System kann durch Polymerisation auf dem Lichtleiterende fixiert werden, so dass sie mit Blutglucose unter Farbänderung irreversibel reagiert. Durch streuende Partikel innerhalb des Chemiesystems wird unter Rückstreuung des über die Lichtleiter 18 eingestrahlten Messlichts eine geräteseitige optische Detektion ermöglicht.

Für eine formschlüssige Antriebskopplung ist das Testelement 10 mit einem das Stechteil 14 tragenden Basisteil 40 versehen. Dieses kann aus Kunststoff bestehen und in einem Zweikomponenten-Spritzgussverfahren an das aus Metall bestehende Stechteil 14 angeformt sein. Denkbar ist es allerdings auch, dass das Stechteil 14 ebenfalls aus Kunststoff besteht und im Bereich des Stechorgans beispielsweise durch eine diamantähnliche Kohlenstoffbeschichtung zusätzlich gehärtet ist.

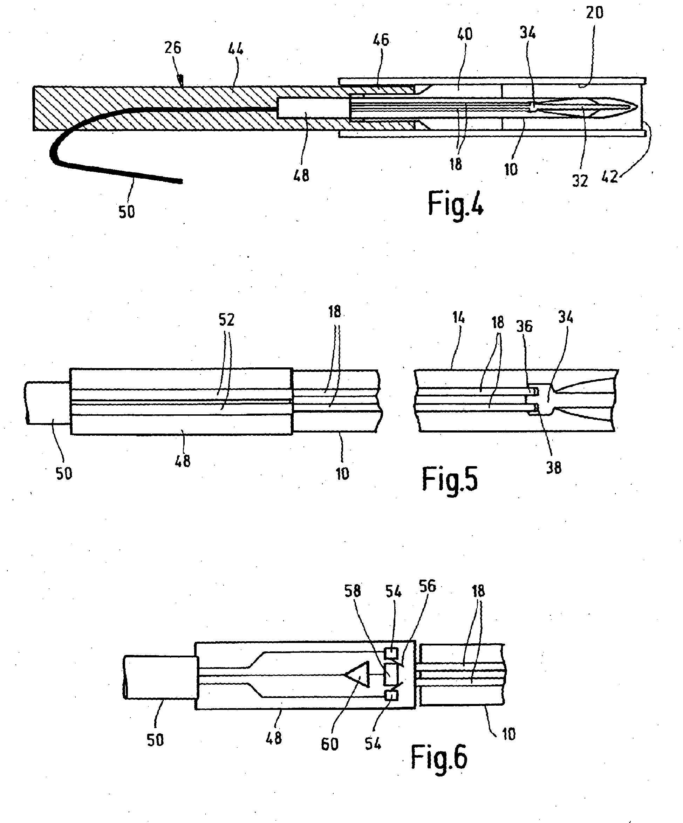

Wie am besten aus Fig. 4 ersichtlich, ist das Stechelement 10 über das Basisteil 40 innerhalb seiner Magazinkammer 20 linear geführt. Eine durchstechbare Siegelfolie 42 an den Enden der Magazinkammer 20 sorgt für einen Steril- und Feuchtigkeitsschutz. Bei der Stechbewegung durchdringt der Antriebstößel 44 die zugewandte Siegelfolie und gelangt mit seinem zangenförmigen Ende 46 mit dem Basisteil 40 in Eingriff. Hierbei werden die Lichtleiter 16 mit einer in dem Stößel 44 angeordneten Schnittstelle 48 lösbar verbunden, wie es unten näher erläutert ist. Die Schnittstelle 48 ist über eine flexible Leitung 50, welche bei der Stechbewegung mitführbar ist, an die Verarbeitungseinheit 27 angeschlossen, so dass dort eine Signalverarbeitung und Auswertung erfolgen kann.

Der Stechvorgang selbst kann in einem gesteuerten Bewegungsprofil eine schnelle Einstichphase, ein vergleichsweise längere, ggf. etwas zurückgezogene Verweil- bzw. Sammelphase und eine wiederum schnelle Rückziehphase umfassen. Dabei ist es möglich, dass auch die Detektion mittels der Lichtleiter 18 mehrstufig abläuft, d.h. dass während der Sammelphase nur ein Blutkontakt erfasst wird, während die eigentliche Analyse erst später in oder nach der Rückziehphase im Gerät 12 erfolgt. Denkbar ist es auch, dass die in dem Schlitz 32 befindliche Blutprobe auf ein gesondertes Testfeld übertragen wird, wie es in der EP 1759633 A1 näher beschrieben ist. In diesem Fall wären die Lichtleiter 18 allein zur Blutkontakterfassung bestimmt.

Fig. 5 zeigt ein Beispiel einer optischen Schnittstelle 48 zur Ankopplung des Testelements 10 über eine optische Steckverbindung, wie sie beispielsweise aus Telekommunikationsanwendungen an sich bekannt ist. Dabei stehen die Lichtleiter 18 des Testelements 10 mit ihren proximalen Enden Stoß auf Stoß in Kontakt mit zugeordneten schnittstellenseitigen Lichtleitern 52, welche über die Flexleitung 50 an ein gerätefestes Sende/Empfangsmodul angeschlossen sind. Es versteht sich, dass die mechanischen Toleranzen der Steckverbindung gering sein sollten, um die erforderliche Übertragungsqualität für das Messsignal zu erhalten.

Um die Toleranzanforderungen zu entschärfen, kann gemäß Fig. 6 eine opto-elektronische Schnittstelle 48 in den Stechantrieb integriert werden. Als Lichtsender sind zwei elektrisch ansteuerbare LED's 54 vorgesehen, die über Blenden 56 auf die Lichtleiter 18 des Testelements 10 ausgerichtet sind. Der Lichtempfänger ist durch eine mittels der Blenden 54 gegen sendeseitiges Übersprechen abgeschirmte Photodiode 58 gebildet, welcher ein Vorverstärker 60 nachgeordnet ist. Auf diese Weise kann ein robustes elektrisches Messsignal aus der beim Stechen mitbewegten Schnittstelle 48 an die Verarbeitungseinheit 27 übermittelt werden.

Möglich ist es auch, mit zwei LED's bei verschiedenen Wellenlängen einzustrahlen, um unterschiedliche Messaufgaben auszuführen (z.B. Erfassung der Testfeldbenetzung getrennt von der eigentlichen Messung).

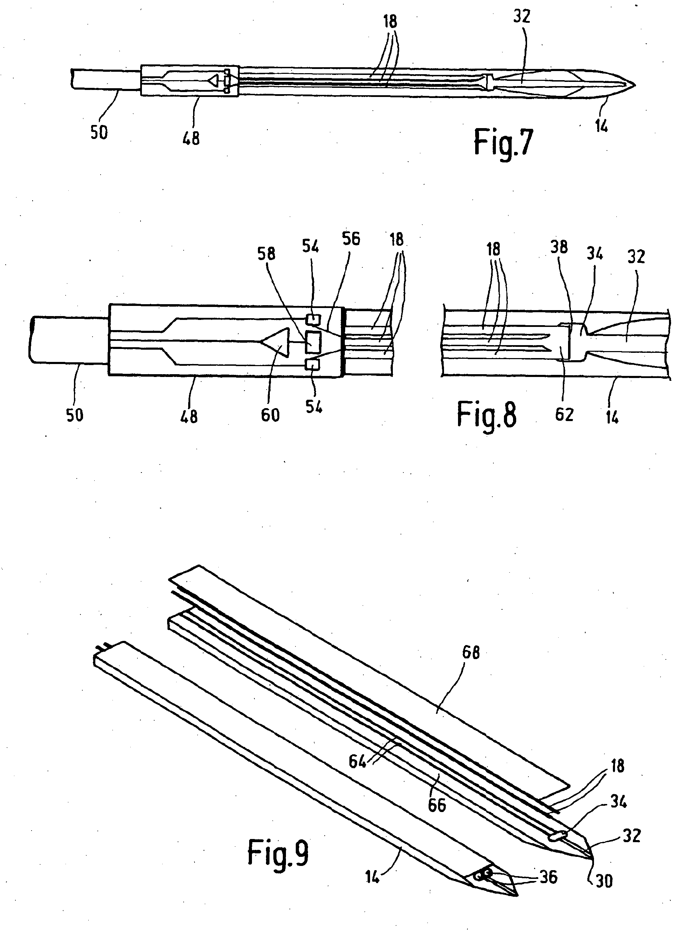

Bei der in Fig. 7 und 8 gezeigten Ausführungsform sind drei parallele Lichtleiter 18 für eine weiter verbesserte Entkopplung zwischen Sende- und Empfangsseite vorgesehen. An der Kopplungsstelle sind die beiden äußeren Lichtleiter für das Sendelicht durch die Blenden 56 von dem Empfänger 58 vollständig abgeschirmt, welcher somit nur auf den Austrittsquerschnitt des mittleren Lichtleiters ausgerichtet ist. In der Messzone 34 hingegen münden die Lichtleiter 18 in ein gemeinsames Endstück 62, welches an seiner freien Stirnseite mit der Testchemie beschichtet ist. Durch die Y-förmige Verbreiterung des mittleren Lichtleiters zu dem Testfeld 38 wird der Empfangsquerschnitt für das Rückstreulicht optimiert. Denkbar wäre es auch, die Leuchtdioden 54 in der Schnittstelle 48 durch Lichtleiter zu ersetzen und nur empfangsseitig eine opto-elektronische Umsetzung in dem bewegten Teil vorzunehmen.

Allgemein ist es für die optische Messanordnung bevorzugt, wenn distalen Endabschnitte der Lichtleiter 18 senkrecht zu dem Testfeld 38 und dieses wiederum senkrecht bzw. quer zu der Fließrichtung der Körperflüssigkeit in der Sammelausnehmung 32 ausgerichtet sind.

Fig. 9 zeigt eine herstellungstechnisch günstige Variante des Stechteils 14 in Form eines Verbundkörpers. Die Lichtleiter 18 sind hierbei in Aufnahmenuten 64 eines Lanzettenteils 66 einsetzbar. Der Lanzettenteil 66 kann aus Edelstahlblech gebildet sein und anschließend an seine Spitze 30 mit der Ausnehmung 32 und der als Durchbruch erweiterten Messzone 34 versehen sein. Die Fixierung der Lichtleiter 18 erfolgt über eine Deckfolie 38, welche auf den Lanzettenteil 66 auflaminiert wird.

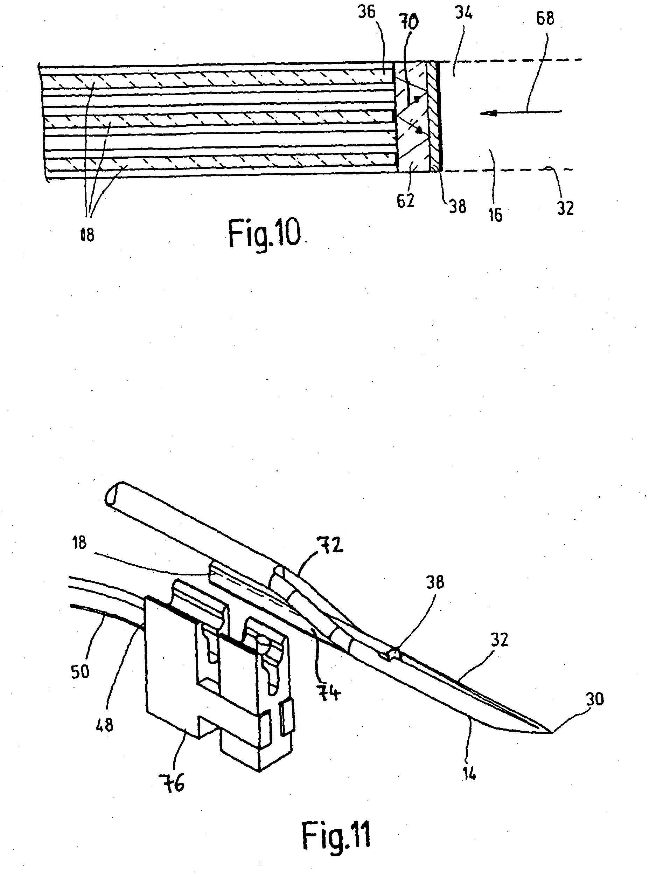

Fig. 10 zeigt eine Ausführungsform ähnlich Fig. 8 mit drei parallel verlaufenden Lichtleitern 18, die zumindest am distalen Endabschnitt mit ihrer Mittellinie bzw. optischen Achse senkrecht zu dem Testfeld 38 ausgerichtet sind. Die optische Ankopplung an die Rückseite des Testfelds erfolgt über eine Trägerfolie 62, die fest mit den Lichtleiterenden verbunden ist, um Reflexionsverluste weitgehend zu reduzieren. Zu diesem Zweck weist die Trägerfolie 62 einen an die Lichtleiter 18 angepassten, d.h. innerhalb weniger Prozent abweichenden Brechungsindex auf. Ebenso ist die auf der freien Vorderseite der Trägerfolie 62 als Testfeld 38 aufgebrachte Reagenzschicht auf einen einheitlichen Brechungsindex an der Grenzschicht zu der Trägerfolie 62 abgestimmt. Dies kann dadurch erfolgen, dass der Brechungsindex durch reaktionsneutrale Zusatzstoffe, beispielsweise Kochsalz in der mit Blutflüssigkeit beaufschlagten Reagenzschicht geeignet angepasst wird. Die Benetzung mit Blut erfolgt dabei über die Testfeldfläche zu einem definierten Zeitpunkt, sobald die in dem Kapillarkanal 32 distal aufgenommene Flüssigkeit aufgrund des kapillaraktiven Transports in Strömungsrichtung 68 frontal auf das zumindest einen Teilquerschnitt des Kapillarkanals proximal blockierende Testfeld auftrifft. Hierfür genügt ein kurzer Fließweg, ohne dass ein Vorbeifließen an einer seitlichen Messfläche erforderlich wäre. Dadurch ist es auch möglich, die sensitive Fläche des Testfelds auf den Strömungsquerschnitt zu reduzieren, so dass nur eine sehr geringe Probenmenge für den eigentlichen Messvorgang benötigt wird und der Flüssigkeitstransport zur Messstelle nicht beeinträchtigt wird. Die optische Anordnung zwischen dem mittleren Einkoppel-Lichtleiter und den beiden seitlich benachbarten Auskoppel-Lichtleitern ist so gewählt, dass über die Trägerfolie 62 ein direkter reflektiver Strahlengang 70 an der Testfeldrückseite möglich ist. Hierfür sollte die Dicke der Folie 62 etwa dem Mittellinienabstand der benachbarten Lichtleiter 18 entsprechen.

Die Ausführungsform nach Fig. 11 zeigt ein Stechelement 14 mit einem abgekröpften Schaft 71, welcher im abgebogenen Bereich geschlitzt ist, um einen Träger für die Signalleiter 18 aufzunehmen. Dieser kann zusammen mit dem Schaft 72 in einen opto-mechanischen Halter eingesetzt werden, wobei eine optische oder elektrische Schnittstelle 48 ankoppelbar ist. Auf diese Weise werden zwei vorzugsweise übereinander liegende Ebenen geschaffen, nämlich eine erste Ebene für die mechanische Aktuation des Stechteils 14 und eine zweite Ebene für die Ein- und Auskopplung des Messsignale. Dies erlaubt eine konstruktive Vereinfachung besonders bei flachen Bauformen beispielsweise für den Einsatz eines Scheibenmagazins mit radialem Ausstoß relativ langer Stecheinheiten 14. Denkbar ist es auch, durch einen abgewinkelten Leitungsträger und einen linearen Stechschaft eine Doppelebene für eine günstige Ankopplung zu schaffen.

Hierbei wie auch in der zuvor erläuterten Ausführungsform ist es möglich, anstelle optischer Signalleiter elektrische Leiterbahnen vorzusehen, welche in Verbindung mit Reagenzschicht-Elektroden eine optimierte Durchführung von an sich bekannten elektrochemischen Glukosemessungen erlauben.

## Patentansprüche

1. Testelement als Einmalartikel zur Untersuchung einer Körperflüssigkeit mit einem in ein Körperteil einstechbaren Stechteil (14), einem daran ausgebildeten Sammelbereich (16) für durch den Einstich erhaltene Körperflüssigkeit und mindestens einem Lichtleiter (18) für eine optische Messung in dem Sammelbereich (16), der als kapillaraktiver Kanal durch eine in Stechrichtung sich erstreckende Sammelausnehmung (16) des Stechteils (14) gebildet ist, wobei der verschiebefest in das Stechteil (14) integrierte Lichtleiter (18) mit seinem distalen Ende (36) in einer proximalen Messzone (34) der Sammelausnehmung (16) angeordnet ist, **dadurch gekennzeichnet dass** die Sammelausnehmung (16) als ein- oder beidseitig offener Schlitz (32) ausgebildet ist, , und dass das distale Ende des Lichtleiters stirnseitig mit einem fest aufgebrachten Testfeld (38) für einen Analyten in der Körperflüssigkeit versehen ist, wobei das Testfeld (38) quer zu der Längserstreckung der Sammelausnehmung (16) ausgerichtet ist, so dass die Körperflüssigkeit frontal gegen das Testfeld (38) anströmt.

2. Testelement nach Anspruch 1, **dadurch gekennzeichnet, dass** der Lichtleiter (18) mit seinem distalen Ende (36) einen Fühler zur Detektion von die Sammelausnehmung (16) füllender Körperflüssigkeit bildet.

3. Testelement nach Anspruch 1 oder 2, **dadurch gekennzeichnet, dass** der Lichtleiter (18) ein Messsignal zur Bewegungssteuerung des Stechteils (14) in Abhängigkeit von einem erfassten Körperflüssigkeitskontakt liefert.

4. Testelement nach einem der Ansprüche 1 bis 3, **dadurch gekennzeichnet, dass** ein auf einen Analyten in der Körperflüssigkeit ansprechendes, irreversibel reagierendes Testfeld (38) vorgesehen ist.

5. Testelement nach einem der Ansprüche 1 bis 4, **dadurch gekennzeichnet, dass** mindestens zwei, vorzugsweise drei gesonderte Lichtleiter (18) für eine Parallelmessung in das Stechteil (14) integriert sind, wobei die distalen Enden (36) der Lichtleiter (18) im seitlichen Abstand voneinander angeordnet sind.

6. Testelement nach Anspruch 5, **dadurch gekennzeichnet, dass** die distalen Enden der Lichtleiter (18) über eine Trägerfolie (62) an ein gemeinsames Testfeld (38) optisch angekoppelt sind, wobei das Testfeld (38) durch eine auf die von den Lichtleitern (18) abgewandte freie Frontseite der Trägerfolie (62) aufgebrachte Reagenzschicht gebildet ist.

7. Testelement nach Anspruch 6, **dadurch gekennzeichnet, dass** die Dicke der Trägerfolie (62) das 0,5- bis 1,5-fache des gegenseitigen Abstands der Mittellinien benachbarter Lichtleiter (18) beträgt.

8. Testelement nach Anspruch 6 oder 7, **dadurch gekennzeichnet, dass** die Lichtleiter, die Trägerfolie (62) und die Reagenzschicht einen aufeinander angepassten, im Wesentlichen übereinstimmenden Brechungsindex aufweisen.

9. Testelement nach einem der Ansprüche 1 bis 8, **dadurch gekennzeichnet, dass** das Testfeld (38) in einer Reagenzschicht reaktionsneutrale Hilfsstoffe, insbesondere Salze wie Kochsalz enthält, welche in Lösung in der Körperflüssigkeit den Brechungsindex der Reagenzschicht an den Brechungsindex des mindestens einen Lichtleiters anpassen.

10. Testelement nach einem der Ansprüche 1 bis 9, **dadurch gekennzeichnet, dass** der Lichtleiter (18) vorzugsweise über eine optische Steckverbindung mit einer optischen Sendeeinheit (54) und/oder Empfangseinheit (58) eines Messgeräts (12) lösbar verbindbar ist.

11. Testelement nach einem der Ansprüche 1 bis 10, **dadurch gekennzeichnet, dass** die Länge der Sammelausnehmung (16) in Stechrichtung gesehen so bemessen ist, dass der proximale Endabschnitt der Sammelausnehmung (16) beim Einstich außerhalb des Körperteils verbleibt.

12. Testelement nach einem der Ansprüche 1 bis 11, **dadurch gekennzeichnet, dass** die Sammelausnehmung (16) ein Volumen von 1 bis 100 nl, vorzugsweise 5 bis 50 nl aufweist.

13. Testelement nach einem der Ansprüche 1 bis 12, **dadurch gekennzeichnet, dass** die Sammelausnehmung (16) einen im Querschnitt verbreiterten proximalen Endabschnitt als Messzone (34) aufweist.

14. Testsystem zur Untersuchung einer Körperflüssigkeit, insbesondere Blut, mit mindestens einem Testelement (10) nach einem der vorhergehenden Ansprüche.

15. Testsystem nach Anspruch 14, **gekennzeichnet durch** ein Magazin, vorzugsweise ein Scheibenmagazin oder Trommelmagazin zur Bevorratung einer Vielzahl von Testelementen (10).

## Claims

1. Test element as a single-use article for examining a body fluid comprising a lancing member (14) that can be inserted into a body part, a collecting area (16) configured thereon for body fluid obtained by the lancing and at least one light guide (18) for an optical measurement in the collecting area (16), which is formed as a capillary-active channel by a collecting recess (16) of the lancing member (14) which extends in the lancing direction, wherein the light guide (18) is integrated into the lancing member (14) such that it is secure against displacement and the distal end (36) thereof is arranged in a proximal measuring zone (34) of the collecting recess (16), **characterized in that** the collecting area (16) is shaped as a longitudinal slot (32) that is open on one or both sides, and that the distal end face of the light guide is provided with a permanently applied test field (38) for an analyte in the body fluid, wherein the test field (38) is aligned transversely to the longitudinal direction of the collecting recess so that body fluid flows frontal against the test field (38).

2. Test element according to claim 1, **characterized in that** the distal end (36) of the light guide (18) forms a sensor for detecting body fluid which fills the collecting recess (16).

3. Test element according to claim 1 or 2, **characterized in that** the light guide (18) generates a measurement signal for controlling the movement of the lancing member (14) depending on a detected body fluid contact.

4. Test element according to one of the claims 1 to 3, **characterized in that** a test field (38) that undergoes an irreversible reaction and responds to an analyte in the body fluid is provided.

5. Test element according to one of the claims 1 to 4, **characterized in that** at least two and preferably three separate light guides (18) are integrated into the lancing member (14) for a parallel measurement, where the distal ends (36) of the light guides (18) are laterally spaced apart.

6. Test element according to claim 5, **characterized in that** the distal ends of the light guides (18) are optically coupled via a carrier foil (62) to a common test field (38), wherein the test field (38) is formed by a reagent layer applied to the free front side of the carrier foil (62) that faces away from the light guides (18).

7. Test element according to claim 6, **characterized in that** the thickness of the carrier foil (62) amounts to 0.5 to 1.5-times the mutual distance between the midlines of neighbouring light guides (18).

8. Test element according to claim 6 or 7, **characterized in that** the light guides (18), the carrier foil (62) and the reagent layer have a matching, essentially identical refractive index.

9. Test element according to one of the claims 1 to 8, **characterized in that** a reaction layer of the test field (38) contains reaction-neutral auxiliary substances and in particular salts such as sodium chloride which when dissolved in the body fluid, adjust the refractive index of the reagent layer to the refractive index of the at least one light guide.

10. Test element according to one of the claims 1 to 9, **characterized in that** the light guide (18) can be detachably connected preferably via an optical plug connection to an optical transmitting unit (54) and/or receiving unit (58) of a measuring device (12).

11. Test element according to one of the claims 1 to 10, **characterized in that** the length of the collecting recess (16) in the lancing direction is such that the proximal end section of the collecting recess (16) remains outside the body part during the lancing.

12. Test element according to one of the claims 1 to 11, **characterized in that** the collecting recess (16) has a volume of 1 to 100 nl, preferably of 5 to 50 nl.

13. Test element according to one of the claims 1 to 12, **characterized in that** the collecting recess (16) has a proximal end section with a widened cross-section as a measuring zone (34).

14. Test system for examining a body fluid, in particular blood, with at least one test element (10) according to one of the previous claims.

15. Test system according to claim 14, **characterized by** a magazine and preferably a disk magazine or drum magazine for storing a plurality of test elements (10).

## Revendications

1. Elément d'analyse sous forme d'article à usage unique pour l'analyse d'un liquide corporel avec une partie d'insertion (14) insérable dans une partie du corps, avec une zone de collecte (16) formée sur celle-ci pour le liquide corporel obtenu par l'insertion, et avec au moins un guide de lumière (18) pour une mesure optique dans la zone de collecte (16), qui est réalisé sous la forme d'un canal à effet capillaire à travers un évidement de collecte (16) de la partie d'insertion (14) s'étendant dans la direction d'insertion, dans lequel le guide de lumière (18) intégré sans glissement dans la partie d'insertion (14) est disposé avec son extrémité distale (36) dans une zone de mesure proximale (34) de l'évidement de collecte (16), **caractérisé en ce que** l'évidement de collecte (16) est réalisé sous la forme d'une fente (32) ouverte sur un côté ou sur les deux côtés, et **en ce que** l'extrémité distale du guide de lumière est pourvue en face frontale d'un champ d'analyse (38) installé en position fixe pour un analyte dans le liquide corporel, dans lequel le champ d'analyse (38) est orienté transversalement à l'extension longitudinale de l'évidement de collecte (16), de telle manière que le liquide corporel arrive frontalement contre le champ d'analyse (38).

2. Elément d'analyse selon la revendication 1, **caractérisé en ce que** le guide de lumière (18) forme avec son extrémité distale (36) un capteur destiné à détecter le liquide corporel remplissant l'évidement de collecte (16).

3. Elément d'analyse selon la revendication 1 ou 2, **caractérisé en ce que** le guide de lumière (18) fournit un signal de mesure pour la commande du mouvement de la partie d'insertion (14) en fonction d'un contact détecté avec du liquide corporel.

4. Elément d'analyse selon l'une quelconque des revendications 1 à 3, **caractérisé en ce qu'**il est prévu un champ d'analyse (38) correspondant, réagissant de façon irréversible à un analyte dans le liquide corporel.

5. Elément d'analyse selon l'une quelconque des revendications 1 à 4, **caractérisé en ce qu'**au moins deux, de préférence trois guides de lumière séparés (18) sont intégrés dans la partie d'insertion (14) pour une mesure parallèle, dans lequel les extrémités distales (36) des guides de lumière (18) sont disposées à distance latérale l'une de l'autre.

6. Elément d'analyse selon la revendication 5, **caractérisé en ce que** les extrémités distales des guides de lumière (18) sont couplées optiquement à un champ d'analyse commun (38) par une feuille de support (62), dans lequel le champ d'analyse (38) est formé par une couche de réactif déposée sur la face frontale libre de la feuille de support (62) située à l'opposé des guides de lumière (18).

7. Elément d'analyse selon la revendication 6, **caractérisé en ce que** l'épaisseur de la feuille de support (62) vaut 0,5 fois à 1,5 fois la distance mutuelle des axes centraux de guides de lumière voisins (18).

8. Elément d'analyse selon la revendication 6 ou 7, **caractérisé en ce que** les guides de lumière, la feuille de support (62) et la couche de réactif présentent un indice de réfraction adapté l'un à l'autre, essentiellement correspondant.

9. Elément d'analyse selon l'une quelconque des revendications 1 à 8, **caractérisé en ce que** le champ d'analyse (38) contient dans une couche de réactif des adjuvants à réaction neutre, en particulier des sels comme du sel de cuisine qui, en solution dans le liquide corporel, adaptent l'indice de réfraction de la couche de réactif à l'indice de réfraction dudit au moins un guide de lumière.

10. Elément d'analyse selon l'une quelconque des revendications 1 à 9, **caractérisé en ce que** le guide de lumière (18) peut être raccordé de façon détachable, au moyen d'une liaison par fiche optique, à une unité d'émission optique (54) et/ou à une unité de réception optique (58) d'un appareil de mesure (12).

11. Elément d'analyse selon l'une quelconque des revendications 1 à 10, **caractérisé en ce que** la longueur de l'évidement de collecte (16), vu dans la direction d'insertion, est dimensionnée de telle manière que la partie d'extrémité proximale de l'évidement de collecte (16) reste à l'extérieur de la partie du corps lors de l'insertion.

12. Elément d'analyse selon l'une quelconque des revendications 1 à 11, **caractérisé en ce que** l'évidement de collecte (16) présente un volume de 1 à 100 nl, de préférence de 5 à 50 nl.

13. Elément d'analyse selon l'une quelconque des revendications 1 à 12, **caractérisé en ce que** l'évidement de collecte (16) présente comme zone de mesure (34) une partie d'extrémité proximale de section transversale agrandie.

14. Système d'analyse pour l'analyse d'un fluide corporel, en particulier du sang, comportant au moins un élément d'analyse selon l'une quelconque des revendications précédentes.

15. Système d'analyse selon la revendication 14, **caractérisé par** un magasin, en particulier un magasin en forme de disque ou un magasin en forme de tambour pour stocker une multiplicité d'éléments d'analyse (10).
